# EUROPEAN PATENT SPECIFICATION

(11) **EP 1 549 330 B1**
(45) Date of publication and mention of the grant of the patent: **02.01.2008**
(21) Application number: 03798838.3
(22) Date of filing: 02.10.2003
(51) Int. Cl.: A61K 31/191

(54) **POMOLIC ACID FOR TREATING MULTIDRUG RESISTANT TUMOURS**
POMOLSÄURE ZUR BEHANDLUNG VON MEHRFACHRESISTENTEN TUMOREN
ACIDE POMOLIQUE POUR TRAITER LES TUMEURS POLYPHARMACORESISTANTES

(30) Priority: 03.10.2002 BR 0204060
(43) Date of publication of application: 06.07.2005
(73) Proprietor: Universidade Federal do Rio de Janeiro, Rio De Janeiro (BR)
(72) Inventor: GATTASS, Cerli, Rocha, Leblon, Rio de Janeiro, RJ (BR); RUMJANEK, Vivian, Mary, Dodd, Humait , Rio de Janeiro, RJ (BR); FERNANDES, Janaina, Olaria, Rio de Janeiro, RJ (BR); CASTILHO, Rachel, Oliveira, Campo Grande, Mato Grosso do Sul (BR); KAPLAN, Maria, Auxiliadora, Coelho, Copacabana, Rio de Janeiro, RJ (BR)
(74) Representative: Fritz & Brandenburg Patentanwälte
(86) International application number: PCT/BR2003/000144
(87) International publication number: WO 2004/030682

(56) References cited:
- DATABASE WPI Derwent Publications Ltd., London, GB; AN 1995-093855, XP002986669 & JP 07 017 995 A (OTSUKA PHARM CO LTD) 20 January 1995
- DATABASE WPI Derwent Publications Ltd., London, GB; AN 1987-296446, XP002986670 & JP 62 209 070 A (OTSUKA PHARM CO LTD) 14 September 1987
- DATABASE WPI Derwent Publications Ltd., London, GB; AN 1983-785487, XP002986671 & JP 58 146 600 A (OTSUKA PHARM CO LTD) 01 September 1983

## Description

### I. Field of the Invention

The present invention is related to the identification of pomolic acid, its isomers and derivatives as anti-neoplastic drugs, to be used for the preparation of a medicament for the treatment of patients suffering from tumours intrinsically multidrug resistant or tumours that acquired this resistance as a result of chemotherapy treatment.

### II. Invention Background

The treatment of human cancer is a branch of medicine that presents still unsolved challenges. Despite the fact that in medicine treatments presenting a greater efficacy and capable of producing the cure of various types of cancer have been developed, these treatments lead many times to undesirable side effects in the patients.

As a result of these problems, new techniques need to be created to solve the existing problems in the present methods. For example, prostate cancer which is the most common cancer in man and responsible for most deaths in this gender, can be treated surgically, using medicaments (chemotherapy/hormones), by radiotherapy or using a combination of these treatments, depending on the stage of the disease.

Among the many existing therapies, chemotherapy is the most promising one for the treatment of the various types of cancer. However, despite the high efficacy of some drugs, the majority of them present serious side effects which preclude their use for prolonged periods of time or repeatedly. For this reason, patents US 5,558,866 and US 5,876,728 propose the use of natural substances, obtained from various plant species, such as plants of the Pittosporaceae family, as the source of new, less toxic chemotherapeutic drugs. That is, drugs with a greater selectivity for the treatment of the tumour resulting in a lesser aggression to the metabolism of the patients' normal cells.

Nowadays, there is several drugs that are used against cancer. Many of them are used alone whereas others show a greater efficacy when used in combination with other drugs or with other therapies.

The patent US 5,602,184 shows the use of some terpenes, chemotherapeutic agent that presents little or even no toxicity, for the treatment of metastatic cancer. This same reference states that treatment with terpenes, selected among acyclic and cyclic monoterpenes, acyclic sesquiterpenes and/or acyclic diterpenes, increases the susceptibility of treated cancer cells to radiotherapy, demonstrating that the combined use of therapeutic methods, despite elevating treatment costs, may produce more promising results for cancer cure.

In more recent studies, the patent US 5,587,402 shows that treatment with limonene, a monoterpene present in the oil of orange skin, have an effect against many types of cancer, such as breast cancer, stomach and lung cancer. However, despite being considered as a non-toxic chemotherapeutic agent for humans in some doses, limonene presents some undesirable side effects, particularly when used in high dosages and short time intervals. As a result of this, their inventors presented another method to produce inhibition or regression of leukaemias, without using limonene, but using instead perillyl alcohol.

In spite of the existence of a great amount of chemotherapeutic agents being produced in the constant struggle against cancer, the efficacy of chemotherapy has been prejudiced by the specific resistance of some tumors to certain chemotherapeutic agents or by the presence in the patients of multidrug resistance, a phenomenon that may be inherent to the patient or acquired as a result of the treatment.

The phenomenon of multidrug resistance (MDR) involves the cross resistance among a number of non-related chemotherapeutic drugs that diverge in respect to their chemical structure, mode of action and cellular target. This group of factors makes MDR one of the main reasons for the chemotherapy failure seen in many tumours.

The MDR phenomenon is multifactorial resulting from defects in the regulation of the genes that control apoptosis, an increase in the process of cellular detoxification, alterations in the DNA repair system and activation or over expression of drug transporter proteins such as P glycoprotein (Pgp), the protein related to multiresistance (MRP) and the protein related to lung resistance (LRP).

Because in MDR, some of the drug transporter proteins function as efflux pumps, removing the chemotherapeutic agent from the cell, one of the strategies utilized nowadays for the treatment of tumors expressing MDR is the use of reversers of the pumps, associated to chemotherapy. The patent US 5,541,232 presents a method for preventing the development of multidrug resistance (MDR) and to revert the existence of multidrug resistance in case it already exists, trough the prevention or correction of the defect in drug accumulation by resistant cells. More particularly this reference describes the administration of MASOPROCOL jointly with anti-neoplasic/cytotoxic drugs that induce the development of multidrug resistance in cells.

The patent EP 941737, despite not approaching the problem of multidrug resistance, presents some inducers of apoptosis in cancer cells. The main point of these products is to inhibit the drug efflux produced by Pgp, as the reduction of the intracellular concentration of the chemotherapeutic agent, is one of the main responsible for the inefficacy of some drugs which would lead to cellular apoptosis in the treatment of cancer. Despite the inhibition of this pump being mentioned in this reference as the cancer treatment *per se,* in the majority of times the use of efflux pump inhibitors in the treatment of tumours presenting multidrug resistance must be associated with a chemotherapeutic agent to produce the desired effect.

Other patents such as US 5,916,566, do also present methods to inhibit the resistance mediated by glycoprotein P (Pgp) to pharmacological compounds by increasing their bio disposal. In this case, cited as a reference, essential oils are utilized to inhibit the activity of P450 or Pgp, which normally are the responsible for the elimination of these compounds.

Taking into account that the MDR phenomenon is one of the main causes of lack of success in tumour chemotherapy, and taking into account that chemotherapeutic agents capable of destroying these types of tumours, avoiding patient's death, are rare, the search for new anti-neoplasic drugs with anti-MDR activity, becomes imperative.

Neto et.all:Cytotoxic Triterpenec Acids from the Peruvian Medical Plant Polylepsis racemosa. Planta Med. 2000,66,483-484 discloses cytotoxicity-guided fraction of the bark and stem extract of Polylepsis racemosa led to the identification of ursolic acid, promolic acid, 3-O-aceteylpomolic acid, and 2-oxopomolic acid. Pomolic acid was the most cytoxic component, and was specific for M-I4 melanoma and ME 180 cervical carzinoma, with GI₅₀ values of 6,9 and 8,3 µg/mL respectively. The document is silent about multi drug resistant cell lines.

### III. Brief description of the invention

A embodiment of this invention is related to the use of pomolic acid for the preparation of medicaments for the treatment of cancer with multidrug resistance.

### IV. Brief Description of the Fgures

Figure 1 describes the inhibition of proliferation of the leukaemic cell line K562 by pomolic acid. K562 cells were treated with 1, 10 and 100 µg / ml of pomolic acid for 48 hours. The values represent the mean ± standard error of three independent experiments.
Figure 2 describes the inhibition of proliferation produced by pomolic acid in the presence of vincristine, on the resistant leukaemia cell line Lucena 1. Lucena 1 cells were treated with 1, 10 and 100 µg / ml of pomolic acid for 48 hours. The values represent the mean ± standard error of three independent experiments.
Figure 3 describes the inhibition of proliferation produced by pomolic acid in the absence of vincristine, on the resistant leukaemia cell line Lucena 1. Lucena 1 cells were treated with 1, 10 and 100 µg / ml of pomolic acid for 48 hours. The values represent the mean ± standard error of three independent experiments.
Figure 4 shows the comparison between figures 2 and 3 in the same graph, comparing the inhibition of proliferation produced by pomolic acid, in the presence or absence of vincristine, on the resistant leukaemia cell line Lucena 1. Lucena 1 cells were treated with 1, 10 and 100 µg / ml of pomolic acid for 48 hours. The values represent the mean ± standard error of three independent experiments.
Figure 5 shows the cytotoxic activity of pomolic acid over the cell line HL-60, another sensitive lineage. HL-60 cells were treated with 1, 5, 10, 25, 50 and 100 µg / ml of pomolic acid for 48 hours. The values represent the mean + standard error of three independent experiments.
Figure 6 shows the cytotoxic activity of pomolic acid over the cell line Caco-2. Caco-2 cells were treated with 1, 5, 10, 25, 50 and 100 µg / ml of pomolic acid for 48 hours. The values represent the mean ± standard error of three independent experiments.
Figure 7 shows the cytotoxic activity of pomolic acid over the Ma104 cell line. Ma104 cells were treated with 1, 5, 10, 25, 50 and 100 µg / ml of pomolic acid for 48 hours. The values represent the mean ± standard error of three independent experiments.
Figure 8 represents the effect of pomolic acid (50 µg/ml) over different cell concentrations of the cell lines Caco-2 and Ma104. Results express the percent inhibition of viability after 48 hours treatment using the acid.

### V. Detailed Description of the Invention

Patients with any kind of tumour, such as those of the ovaries, breast, lung, colon and many others, may eventually develop MDR.

The MDR phenomenon has been associated to the over expression of MDR genes that code for transporter proteins expressed in the plasma membrane, such as the glycoprotein P (MDR-1 gene) and the protein of multidrug resistance - MRP (MRP-1 gene). The expression of these genes is associated with a reduced cellular concentration of drugs, resulting from an energy-dependent active efflux mechanism of chemical compounds, as seen with Pgp, or of a mechanism dependent of the conjugation of these compounds with glutathione, as seen with MRP. Other genes also associated to the MDR phenomenon, code for proteins expressed in intracytoplasmatic vesicles, such as lung resistance protein - LRP, that is implicated in nuclear-cytoplasmic trafficking and in this way confers resistance to DNA-binding drugs. The great importance of these proteins to the phenomenon of multidrug resistance in tumour cells makes compounds, capable of modulating their activity, in powerful drugs for the treatment of cancer with this type of resistance.

The multiple resistance induced by the over expression of Pgp at the plasma membrane may be reversed *in vivo* and *in vitro* by a variety of hydrophobic, structurally and functionally unrelated substances, known as MDR reversers, modulatory agents or chemosensitizers. These substances block the efflux pump allowing the intracellular accumulation of the chemotherapeutic agent. The first substances utilized as reversers were the calcium channel inhibitor verapamil, the immunossupressor cyclosporin A and a group of substances with known activity in other biological systems and totally unrelated among themselves such as phenotiazines , antimalarials, antibiotics etc.

Second- and third-generation modulators are already being produced and being evaluated regarding their reversal capacity. Some of these agents, are now being used in patients in combination with chemotherapics with variable toxicity profiles. The toxicity observed derives from the pharmacological action of the drug being used as a reverser or from the fact that Pgp is also expressed in some normal tissues where the physiological role of this protein is unknown. Therefore, new chemotherapeutic agents that are not substrates for MDR transporters or that could function as reversers have a very high pharmaceutical interest.

However, the availabe pump reversers, do not show efficacy for all kinds of MDR tumours. This occurs because technically each type of cancer (leukaemia, breast, colon, lung and others) may respond in a different way to the chemotherapic.

Therefore, some patients bearing the same types of cancer, may eventually express the MDR (multidrug resistance) phenotype either intrinsically or as a result of the patient treatment with the chemotherapic. As the MDR phenomenon involves cross-resistance to structurally unrelated drugs that differ in their structure, mode of action and cellular target, treatment of these patients with the know chemotherapeutic drugs are totally without efficacy. In this case the patient will dye unless a drug is identified, capable of circumventing or inhibiting the mechanisms activated by the MDR phenotype and in this way kill the cell.

In this way, the identification of a new drug with anti-MDR properties, especially if this drug is not a substrate for the transporter proteins, will always be of great clinical relevance, as it may represent the only alternative of cure for patients that showed resistance to the chemotherapeutic drugs available.

The present invention represents the offer of the use of pomolic acid as drug for the treatment of tumours expressing MDR and another alternative of treatment for patients bearing this type of tumour without the need of other drugs or additional therapies.

The fact that the drug has a direct effect over the tumour, avoids the need for the association of reversing substances, decreasing the risk of possible secondary effects that they may cause to the patient and being able to reduce treatment costs.

The substance used in the present invention is a terpene, more specifically a triterpene, obtained from natural sources, such as plants, or obtained by chemical synthesis.

The triterpenes belong to a big family of compounds known as cycloesqualenoids, derived from the secondary metabolism of plants. In the last years many biological activities have been attributed to compounds that belong to this class, including anti-fungal, anti-inflammatory, anti-HIV and more recently anti-tumour activities.

When studying this class of substance, through the screening of anti-tumour agents, we observed that a reasonable number of bioactive compounds could be identified with anti-proliferative and cytotoxic activity for tumour cell lines; such as Taxol, terpenes, Yamagishi e col. 1988; Kim e col. 1998, 2000, betulinic acid, a triterpenoid isolated from the bark of Physocarpus intermedius, has been characterized as a potent tumoricidal agent inducing apoptosis in tumours of neuroectodermic origin, Schmidt et al. 1997; Fulda et al., 1997, 1998, 2001 and acting over melanomas *in vitro* and *in vivo,* Kim et al., 2000; Pisha et al., 1995; Raisova et al., 2001; and oleanoic acid, another triterpene isolated from the same plant, inhibits the growth of tumour cell lines *in vitro,* Kim et al., 2000, the angiogenesis, Sohn et al., 1994, and the development of tumours induced by TPA, Tokuda et al., 1996. Most recent works established that, in the same way as betulinic acid, other triterpenes are also capable of inducing the process of programmed cell death in tumour cells, Konopleva et al., 2002.

However, contrary to what one may believe, despite the great number of studies already carried out, the present inventors have been capable of recognizing among the triterpenes, more specifically in pomolic acid and its derivatives, a biological activity never tested before.

Therefore, despite the cytotoxic activity of this triterpene in melanoma cell lines had been already described in the literature, Neto et al., 2000, data presented in this document demonstrate the cytotoxicity of pomolic acid on cells that express the MDR phenotype. This represents a great advance in the capacity to kill tumors that are resistant to conventional chemotherapies.

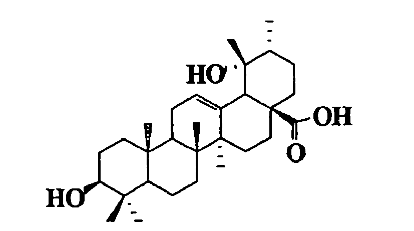

As mentioned above, pomolic acid is a molecule derived from plants and well know in the literature.

In this way, the identification of the active substance of this invention as being pomolic acid was easy, using for this studies of magnetic ressonance already present in state of tecnique by Maillard M, Adwunmi CO, Hostettman K. A triterpene glycoside from the fruits of Tetraplura tetraplera. Phytochemistry 1992, 31(14), 1321-1323: Kakuno T, Shikawa KY, Arihara S. Triterpenoid saponmins from Ilex crenata fruuit. Phytochemistry 1992; 31(10, 3553-3557. In this way, we may assure that pomolic acid, according with the present invention, is the substance responsible for the citoxicity on cancer cells presenting resistance to multiple drugs.

Therefore, this invention presents the use of pomolic acid, its isomers and derivatives as a substance with anti-MDR properties, capable of eliminating cancer cells and with low or no toxicity to the patients' normal cells.

### VI. Examples for Illustration

With the objective of obtaining, testing and proving the efficacy of pomolic acid, its isomers and derivatives as a chemotherapeutic drug with anti-MDR activity, many studies were realized as shown below.

### Example 1: Method for obtaining pomolic acid.

a. Summary of the methodology: Pomolic acid was obtained through the successive extraction of leaves with organic solvents, followed by the fractionation of the extract in silica gel and amberlite columns. The purity was characterized through the measurements of CG/MS, 1H and 13C NMR, the point of fusion and optical rotation and its identification was performed comparing the physical and spectral data with that of the triterpenes already described in Maillard et al., 1992; Kakuno et al., 1992; Mahato et al., 1994.
b. Detailed methodology: Methanolic extracts of the dry and lyophilized leaves of Crysobalanus icaco L. were successively fractionated with hexane, CH2Cl2, AcOEt and BuOH. Pomolic acid was obtained from the CH2C12 fraction of the leaves of Chrysobalanus icaco L. through the fractionation in silica gel column followed by elution in a mixture of hexane/ ethyl acetate (50%). This fraction was re-chromatographed in Amberlite ZAD-2 and the elution with methanol resulted in a white solid that presented the profile of a pure substance in thin layer chromatography. The purity of the compound was corroborated through the measurements of CG/MS, 1H and 13C NMR, the point of fusion and optical rotation. The compound was identified as pomolic acid by comparison with the physical and spectral data of triterpenes already described in Maillard et al., 1992; Kakuno et al., 1992; Mahato et al., 1994.

### Example 2: Evaluation of the inhibition of cellular proliferation

As tumours grow spontaneously, one of most common methods to test the anti-tumour activity of a drug is to estimate the effect of the drug on the inhibition of cell proliferation. For these studies sensitive or multidrug resistant cell lines can be used.

The inhibition of cellular proliferation was measured through the incorporation of radioactive thimidine (3H-TdR). For this, 180 µl of the cell suspensions are delivered in wells of a 96 wells culture microtiter plate, 2x10⁴cells/well, followed by incubation in a CO₂ incubator, at 37°C for 24h. After this period, 20µl of RPMI (control) or the drug to be tested at the concentrations of 100µg/ml, 50µg/ml, 25µg/ml, 10µg/ml and 1µg/ml, are added to each group of four wells. Some wells, to be used as controls, receive 20 µl of DMSO in the same concentrations carried by the drugs. Eight hours before the end of the culture, the plate received a pulse of radioactive thymidine (1µCi/well) and radioactivity was measured in a β counter. The percentage of inhibition was calculated taking as a reference cell growth in the presence of DMSO.

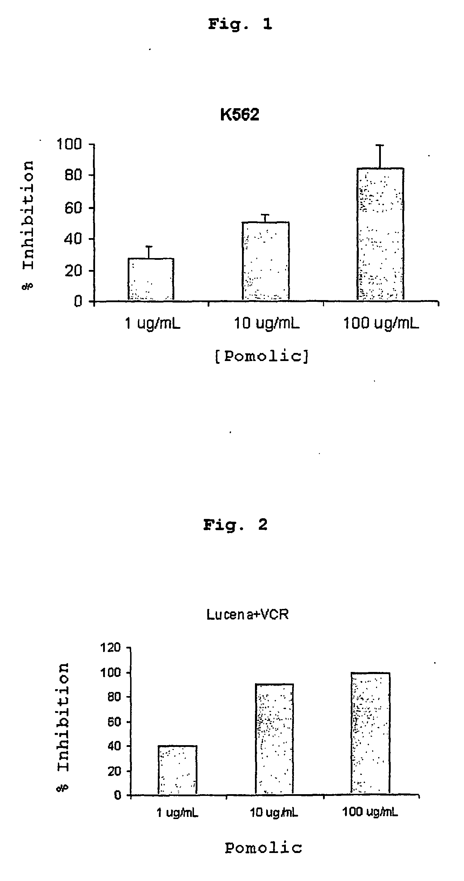

These results are demonstrated in figures 1 to 3, showing that pomolic acid inhibits both the proliferation of sensitive cell lines (K562) and multidrug resistant cell lines (Lucena), and that its effect is dose-dependent.

### Example 3: Evaluation of cytotoxicity

One of the characteristics wanted from a chemoterapeutic drug is its capacity of killing a tumour cell.. This activity may be estimated by the quantification of the number of dead cells or the number of cells that remain viable after treatment. Measurements of DNA fragmentation indicate the mechanism (necrosis or apoptosis) through which the drug is acting.

The evaluation of the capacity of pomolic acid, its isomers and derivatives of killing tumour cells will be performed *in vitro* by the MTT method. The MTT method (3-4,5-dimethylthiazol-2yl) -2,5-diphenyltetrazolium bromide), that is based on the reduction of this compound to Formazam by the enzime NADH dehydrogenase, Mosmann 1983, allows the quantification of the number of viable cells after treatment. For this, cells from the different cell lines will be processed as described above, distributed into plates and treated with the drug desired concentrations (1, 5, 10, 25, 50 or 100µg/ml) or DMSO. After 48h, 20µl of MTT (5mg/ml) will be added to each well and the plate will be incubated for a further 4h in an incubator at 37°C. Following centrifugation, the formazan cristals will be dissolved with DMSO (200µl/well) and the reading of the absorbance will be made in an ELISA reader at 570nm wavelenght. The percentage of inhibition of cellular viability will be calculated using as reference cells treated with DMSO.

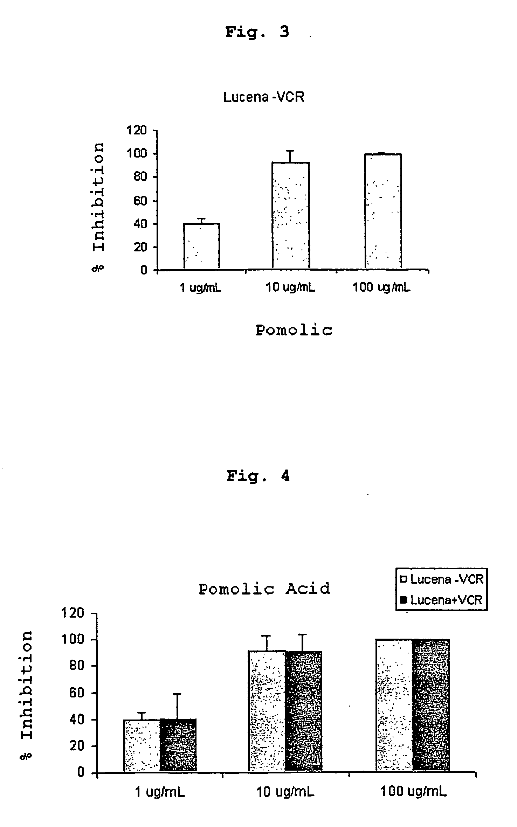

The results presented in figure 4 show that the toxicity of pomolic acid grows as the dose increases. These results also showed the efficacy of the anti-tumour activity of pomolic acid over leukaemic cell lines other than K562 and Lucenal.

### Example 4: Evaluation of apoptosis induction

A large number of chemotherapeutic drugs lead tumour cells to death through the induction of apoptosis. Measurements of DNA fragmentation indicate the mechanism (necrosis or apoptosis) through which the drug is acting.

The effect of pomolic acid over DNA fragmentation was studied through the analysis of the cell cycle by flow cytometry. For this 90 µl of cells (2.5x10⁵ cells/well) will be distributed in 96 well plates to which it will be added 10µl of medium, DMSO or the drugs under test (10, 25, 50 and 100µg/ml). After the defined period of time the cells will be harvested and spun down at 240g for 5 min. The pellet will be ressuspended in 300□1 of a HFS solution (HFS: 0.1% of Triton x-100, 0.1% of sodium citrate and 50µg/ml of propidium iodide) and incubated at 4°C for 1h, in the absence of light. The samples will be read in a flow cytometer (Becton and Dikinson) using the FL2 channel.

Table 1 shows that treatment with pomolic acid produces DNA fragmentation in the cells. The K562 cells were treated with 10, 25, 50 and 100 µg/ml of pomolic acid for 18h, lysed by HFS treatment (that contains propidium iodide) and DNA fragmentation was measured by FACS (flow cytometry). The values represent the mean ± SD of three independent experiments. Table 1. Percentage of DNA fragmentation in K562 cells treated with pomolic acid.

| | Drug concentration (µg/ml) | | | |
|---|---|---|---|---|
| Treatment | 10 | 25 | 50 | 100 |
| *control | 5.08 ± 0.25 | 5.05 ± 0.27 | 4.91 ± 0.20 | 5.26 ± 1.09 |
| pomolic | 4.31 ± 1.21 | 13.72 ± 0.52 | 29.93 ± 2.74 | 74.88 ± 5.69 |

| | | | | |
|---|---|---|---|---|
| *Cells were treated with DMSO in the same concentrations carried by the drug dilution. Results represent the mean ± SD of 3 experiments. | | | | |

DNA fragmentation described in table 1, demonstrates that the cytotoxic effect of pomolic acid is mediated by apoptosis induction.

### Example 5: Estimation of the reverser activity of pomolic acid

A possible reverser activity of pomolic acid was evaluated by testing its effect over Lucena 1 cell line, that over expresses P glycoprotein (Pgp).

Table 2 demonstrates the resistance of Lucena 1 cells and the sensitivity of K562 cells to the chemotherapeutic agent Vincristine, a drug largely utilized in cancer treatment. Rumjanek et al. (2001) have shown the resistance of Lucena 1 to other chemotherapeutic agents characterizing it as a MDR cell line.

**Table 2. Effect of vincristine (VCR) on the growth of K562 and Lucena 1. Results express the percentage of survival in relation to the untreated control.**

| Treatment | K562 (%) | Lucena (%) |
|---|---|---|
| Control | 100 | 100 |
| VCR 60nM | 4.9 + 3.1 | 97.8 + 3.7 |
| VCR 30nM | 10 + 2.9 | 99.5 + 6.5 |
| VCR 15nM | 29.7 + 7.7 | 97.4 + 6.3 |
| VCR 7.5nM | 70.1 + 11.8 | 98.4 + 1.9 |
| VCR 3.75nM | 76 + 7.8 | 106.6 + 3.8 |

Table 3 shows that the use of modulators of the efflux pump, such as cyclosporin A (CSA) and verapamil (VP) do not affect Lucena's growth but are necessary to guarantee the efficacy of the chemotherapeutic agent vincristine (VRC) over this cell line. This result indicates that Lucena 1 resistance is mediated by Pgp.

**Table 3 Modulatory effect of cyclosporin A (CSA) and verapamil (VP) on the growth of K562 and Lucena 1 cells. Results express the percentage of survival.**

| Treatmento | K562 (%) | Lucena (%) |
|---|---|---|
| Controle | 100 | 100 |
| VCR 60 nM | 3.1 ± 1.5 | 105.3 ± 7.3 |
| CSA 160 nM | 101.8 ± 3.4 | 105.2 ± 5.3 |
| CSA/VCR | 1.8 ± 0.9 | 2.6 ± 1.4 |
| VP 5 µM | 106.3 ± 2.2 | 103.5 ± 3.0 |
| VP/VCR | 2.9 ± 0.7 | 3.6 ± 1.6 |

To test if pomolic acid acted as a Pgp reverser its effect on the growth of Lucena 1 was evaluated in the presence and absence of the chemotherapeutic agent vincristine. The experimental design was the same one as described in example 1.

Data of figure 2, showing that pomolic acid inhibits Lucena's 1 growth in the presence of vincristine, could suggest a modulatory action over Pgp similar to that of CSA and VP shown on Table 2. However, the fact that the acid kills Lucena 1 in the absence of vincristine, according to figure 3, shows that this acid is not a Pgp substrate excluding a possible modulatory role and demonstrating its capacity of acting directly on MDR cells.

Therefore, in addition to anti-tumor activity on sensitive cell lines, the data above puts into evidence the potential of pomolic as a potent anti-MDR agent. The results also highlight a decrease in possible undesirable impacts over the body, as treatment with pomolic acid does not require the use of modulatory agents, which, in general, enhance the side effects of the chremotherapeutic drug.

### Example 6:Pomolic's acid activity over the tumor lines with the MDR phenotype.

Pomolic' s acid activity was evaluated on cells sensitive to chemotherapeutic agents, such as leukaemic lines (K562 and HL-60), of lung cancer (A549) and throat (HEp-2), and on multidrug resistant of leukaemic origin (Lucena 1), of colon cancer (CaCo-2) and of monkey kidney (MA104). The resistance of Lucena 1 is attributed to the expression of Pgp whereas the resistance of CaCo-2 and MA104 is attributed to Pgp and other MDR genes.

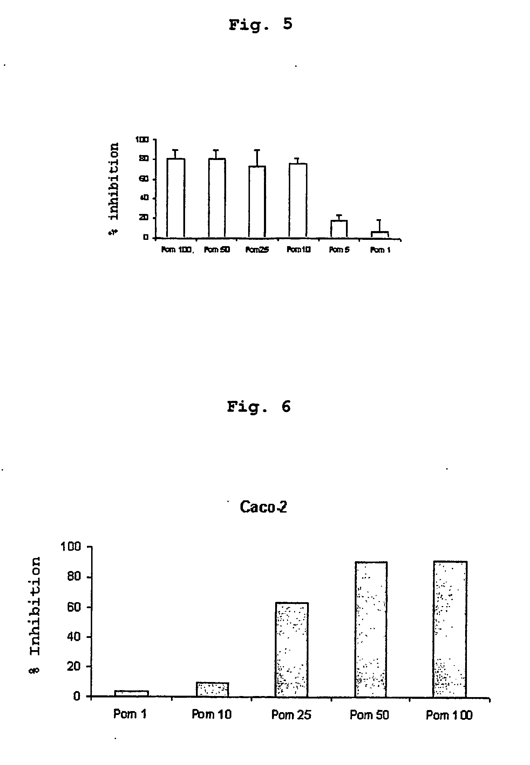

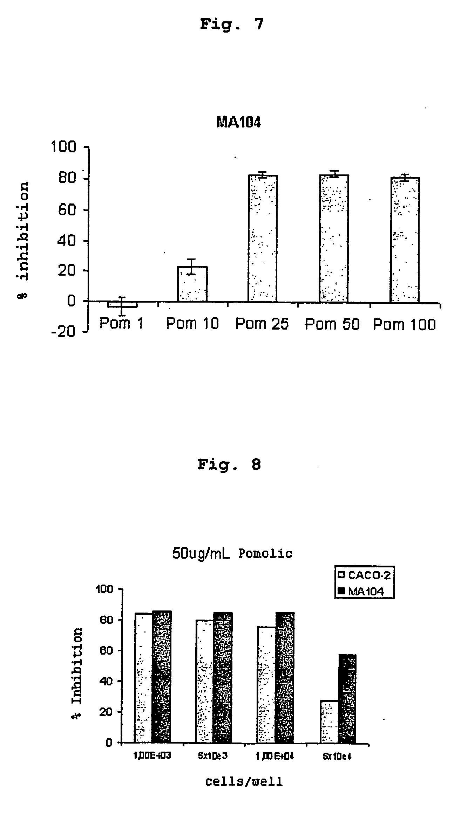

For this 180 µl of the cell suspension (CaCo-2 or MA104) will be delivered to wells in a 96 wells microtiter plate, 2x10⁴ cells/well, and the plate incubated in a CO₂ incubator at 37° C for 24 h. After this period of time, the cells will be treated with different concentrations of the drug (1, 5, 10, 25, 50 and 100µg/ml) or of DMSO, incubated for a further 48 h, treated with 20µl of MTT (5 mg/ml) and processed as described in example 2. The results of figures 6 and 7 express the percentage of inhibition of viability of CaCo2 and MA104 after 48 h treatment with the acid. Figure 8 presents the effects of 50µg/ml of pomolic acid over different cells concentrations of these cell lines. These results demonstrate that, not only pomolic acid is capable of directly killing Pgp expressing cells, but it is also of exerting a potent tumoricidal effect on resistant cell lines that express products of other genes capable of inducing MDR.

In conclusion, figure 1 shows that pomolic acid inhibits the growth of leukaemic K562 cells (chronic myeloid leukaemia) and figures 2 and 3 show the effect of pomolic acid inhibition on Lucena 1 cells (MDR cell line). The fact that pomolic acid inhibits Lucena's growth in the presence of vincristine, shown in figure 2, could suggest a modulatorny action over Pgp similar to the one of CSA or VP shown in Table 2. However, the fact that the acid is capable of killing Lucena 1 in the absence of vincristine, as shown in figure 3, shows that this acid is not a Pgp substrate, excluding a possible modulatory role and demonstrating its chemotherapeutic activity on MDR cells.

In addition to acting on K562, pomolic acid shows a cytotoxic activity towards other sensitive cell lines such as HL-60 (acute myeloid laeukemia), A549 (lung cancer) and HEp-2 (throat cancer), and also resistant ones that express other genes capable of producing MDR through mechanisms other than Pgp such as CaCo-2 (colon cancer) and MA104 (monkey kidney cancer). Pomolic' s capacity of killing these two resistant cell lines is shown in figures 6, 7 and 8.

For therapeutic applications against multidrug resistant tumors, in accordance with this invention, a therapeutically efficacious amount of pomolic acid, its isomers or derivatives, is administered, orally or systemically. This efficacious amount being comprised preferentially between 0.01 mg/kg and 100 mg/ kg body eight.

The chemical composition utilized in this invention contains a sufficient efficacious amount of pomolic acid, its isomers or derivatives and pharmaceutically acceptable vehicles for systemic or oral administration. The pharmaceutically acceptable vehicles consist of a organic solvent, further diluted in a saline solution or another equivalent isotonic solution. The solvent may be dimethylsulfoxide or another organic solvent with acceptable use in humans, diluted in saline solution.

The method to prepare this pharmaceutical composition consists in a first step of solubilization in dimethylsulfoxide or another solvent pharmaceutically acceptable for use in humans and a posterior dilution in a saline solution, in such a way the concentration of dimethylsulfoxide does not exceed 1%, and in this way eliminating the toxic effects of dimethylsulfoxide.

## Claims

1. Use of a composition comprising at least one pomolic acid, its isomers or derivatives and a vehicle for the manufacture of a medicament for the treatment of multidrug resistant tumors.

2. The use according to claim 1 wherein, said pomolic acid is (I):

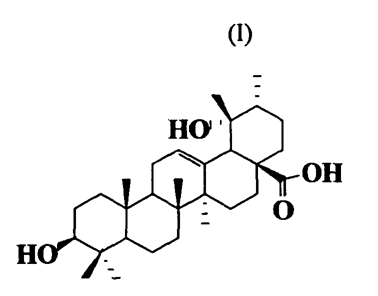

3. The use according to claim 1 wherein said vehicle is a pharmaceutical accepted vehicle for systemic or oral administration.

4. The use according to claim 1 wherein said vehicle is an organic solvent, diluted solution or another equivalent isotonic solution.

5. The use according to claim 4 wherein the organic solvent is dimethylsulfoxide or other organic solvent acceptable for humans, diluted in a saline solution.

6. The use according to claim 1 wherein, said that pomolic acid, it isomers and/or derivatives are in the concentration range from 0,1% to 100% in weight.

7. The use according to claim 6 wherein said that pomolic acid, isomers or derivatives are in the concentration range from 10µg/mL to 100µg/mL.

## Patentansprüche

1. Verwendung einer Zusammensetzung umfassend wenigstens eine Pomolsäure, deren Isomere oder Derivate und ein Vehikel für die Herstellung eines Medikaments für die Behandlung von gegen Arzneimittel mehrfach resistenten Tumoren.

2. Verwendung nach Anspruch 1, bei der diese Pomolsäure diejenige mit der nachfolgenden Formel (I) ist:

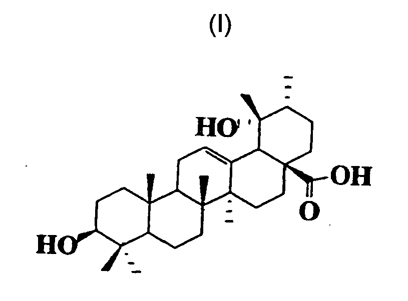

3. Verwendung nach Anspruch 1, worin das Vehikel ein pharmazeutisch akzeptables Vehikel für die systemische oder orale Verabreichung ist.

4. Verwendung nach Anspruch 1, bei der das genannte Vehikel ein organisches Solvenz ist, eine verdünnte Lösung oder eine andere äquivalente isotonische Lösung.

5. Verwendung nach Anspruch 4, bei der das organische Solvenz Dimethylsulfoxid ist oder ein anderes organisches Solvenz, welches für den Menschen akzeptabel ist, verdünnt in einer Salzlösung.

6. Verwendung nach Anspruch 1, bei der die genannte Pomolsäure, deren Isomere und/oder Derivate in einem Konzentrationsbereich on 0,1 Gew.% bis 100 Gew.% vorliegen.

7. Verwendung nach Anspruch 6, bei der die genannte Pomolsäure, deren Isomere oder Derivate in einem Konzentrationsbereich von 10 µg/mL bis 100 µg/mL.

## Revendications

1. Utilisation d'une composition comprenant au moins un acide pomolique, ses isomères ou dérivés et un véhicule pour la production d'un médicament pour le traitement des tumeurs polypharmacorésistantes.

2. Utilisation selon la revendication 1, dans laquelle l'acide pomolique est celle de la formule (I) suivante :

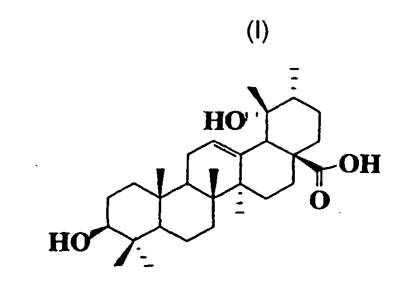

3. Utilisation selon la revendication 1, dans laquelle ledit véhicule est un véhicule acceptable pour l'administration systémique ou orale.

4. Utilisation selon al revendication 1, dans laquelle ledit véhicule est un solvant organique, une solution diluée ou d'autre solution équivalente isotonique.

5. Utilisation selon la revendication 1, dans laquelle le solvant organique est le sulfoxide diméthyl ou d'autre solvant organique acceptable pour l'homme, dilué dans une solution saline.

6. Utilisation selon la revendication 1, dans laquelle ledit acide pomolique, ses isomères ou dérivés ont une concentration dans une gamme de 0,1 % de poids jusqu' à 100 % de poids.

7. Utilisation selon la revendication 1, dans laquelle l'acide pomolique, ses isomères ou dérivés ont une concentration dans une gamme de 10 µg/mL jusqu' à 100 µg/mL.
